# EUROPEAN PATENT SPECIFICATION

(11) **EP 2 907 477 B1**
(45) Date of publication and mention of the grant of the patent: **12.06.2019**
(21) Application number: 13842341.3
(22) Date of filing: 10.09.2013
(51) Int. Cl.: A61B 5/107

(54) **METHOD AND DEVICE FOR DISPLAYING THREE-DIMENSIONAL SIMULATION OF UPPER AND LOWER TOOTH ROWS**
VERFAHREN UND VORRICHTUNG ZUR ANZEIGE EINER DREIDIMENSIONALEN SIMULATION VON OBEREN UND UNTEREN ZAHNREIHEN
PROCÉDÉ ET DISPOSITIF D'AFFICHAGE DE SIMULATION TRIDIMENSIONNELLE DE RANGÉES DE DENTS SUPÉRIEURE ET INFÉRIEURE

(30) Priority: 26.09.2012 JP 2012212166
(43) Date of publication of application: 19.08.2015
(73) Proprietor: KABUSHIKI KAISHA SHOFU, Kyoto-shi, Kyoto 605-0983 (JP)
(72) Inventor: SHIGEMOTO, Shuji, Tokushima-shi Tokushima 770-8503 (JP); SATSUMA, Toyoko, Tokushima-shi Tokushima 770-8503 (JP); NOGUCHI, Naoto, Tokushima-shi Tokushima 770-8503 (JP); SUZUKI, Yoshitaka, Tokushima-shi Tokushima 770-8503 (JP); ISHIKAWA, Teruaki, Tokushima-shi Tokushima 770-8503 (JP); OKURA, Kazuo, Tokushima-shi Tokushima 770-8503 (JP)
(74) Representative: Eisenführ Speiser
(86) International application number: PCT/JP2013/074418
(87) International publication number: WO 2014/050543

(56) References cited:
- JP-A- H0 678 937
- JP-A- H09 238 963
- JP-A- 2001 517 480
- JP-A- 2007 236 637
- JP-A- 2010 507 446
- JP-B2- 4 612 915
- US-A1- 2002 048 741

## Description

### TECHNICAL FIELD

The present invention relates to an apparatus for three-dimensional simulation display of upper and lower tooth rows at a high speed. The present invention particularly relates to an apparatus for measuring three-dimensional shapes of upper and lower tooth rows quickly, wherein, preferably, occlusion of the upper and lower tooth rows can be determined at a high speed based on the three-dimensional shapes of the upper and lower tooth rows thus measured.

### BACKGROUND ART

In dental examination and treatment, bite of upper and lower teeth (occlusion) very often has to be accurately recorded and diagnosed, but, in reality, dentists decide on courses of treatment depending on their experiences.

As a diagnostic examination method of occlusion which is used in clinical dentistry, occluding paper is frequently used due to its convenience, and also black silicone, wax or the like for diagnostic examination of occlusion are sometimes used. Recently, occlusal states have been able to be quantatively evaluated by using a T-scan system (Non Patent Literature 1) and a dental pre-scale (Non Patent Literature 2). However, since in these diagnostic examination methods, occluding paper, a special sheet or the like need to be inserted between upper and lower teeth, only "stationary" occlusion contact relations can mostly be determined, and it is difficult to determine a "dynamic" occlusion contact relation at a time of functioning, such as mastication or the like, and at a time of manifestation of bruxism that is considered as a developing factor or an aggravating factor of temporomandibular disorders.

Further, at present, in a dental technical industry, very frequently, tooth row shapes are digitized, and technical work is digitally performed. However, in current systems, the technical work is digitized without precisely determining the occlusal position at present, and the occlusal position thus obtained has an inaccurate relationship with an actual occlusal position. Further, since jaw movement data are not available, current systems have the problems such that proficient production of occlusal surfaces are difficult.

### CITATION LIST

### PATENT LITERATURE

[Patent Literature 1] JP4324386B
[Patent Literature 2] JP 4612914 B
[Patent Literature 3] JP 4612915 B
[Patent Literature 4] JP4665051B

US 2002/048741 discloses an apparatus for creating a dental model by using digital images of a patient's upper and lower dental arches, comprising position sensors for recording motion of the mandible, a bite impression provided with scales and coated with impression material, means for detecting the shape of the upper and lower dental arches.

### NON PATENT LITERATURE

[Non Patent Literature 1] "Occlusal Contact Examination Device T-Scan III", [online], Nitta Corporation, [Search September 10, 2012], The Internet <URL: http://www.nitta.co.jp/images/product/pdf/tactile_system/t-scan3_201104.pdf>
[Non Patent Literature 2] "Pressure Measurement Film (Prescale)", [online], Fujifilm Corporation, [Search September 10, 2012], The Internet <URL: https://fujifilm.jp/business/material/prescale/promotion/index.html>
[Non Patent Literature 3] Suzuki Atsushi, "A Study on Mandibular Movement in Six Degree-of-freedom with a Newly Developed Jaw Movement Analyzer", J Jpn Prosthodont Soc., 31:712-725, 1987
[Non Patent Literature 4] Ohkubo Yukiko, "Kougousesshoku no Sanjigenkaisekisisutemu no Kaihatsu", J Jpn Prosthodont Soc., 36:53-63, 1992

### SUMMARY OF INVENTION

### TECHNICAL PROBLEM

The precision which a living body requires to an occlusal surface form is considered to be approximately 20 µm. In inspection, examination and diagnosis, and determination of an effect of treatment of a dynamic state of occlusion which requires high precision as described above, an occlusion visualizing technique is indispensable. However, the method capable of observing dynamic occlusal contact (an occlusion visualizing technique) while functioning, such as mastication or the like, with sufficient precision with no insertion between the occlusal surfaces is not established yet. The occlusion visualizing technique mentioned here refers to the integration of (a) a highly precise six-degrees-of-freedom jaw movement measuring technique, (b) a high-precision three-dimensional shape measuring technique, (c) a technique of superimposing the jaw movement data and tooth row shape data, and (d) a technique of systemizing easy visualization of the above (simulation display).

Among these techniques, concerning (a) the highly precise six-degrees-of-freedom measuring technique for movement, there exist techniques provided by the inventors of the present application and others (Patent Literature 1 to Patent Literature 4). According to the techniques, relative movement of the upper jaw and the lower jaw (normally, the upper jaw is set as the reference, and the movement of the lower jaw is observed in six degrees of freedom relative to the upper jaw) can be measured and the movement data is produced.

The high-precision three-dimensional shape measurement of tooth rows in (b) has been conventionally performed by the method as follows.
(1) An impression material such as a dental silicone material is pressed to the upper tooth row, and a female mold is obtained.
(2) Based on the female mold, a gypsum model of the upper tooth row is produced.
(3) The three-dimensional shape of the gypsum model is measured, and the three-dimensional data at respective points on the surface of teeth of the upper tooth row is acquired.

The above is similarly performed for the lower tooth row.

Conventionally, as above, the three-dimensional shape data is acquired based on gypsum models and therefore, when a contact type three-dimensional shape measuring instrument(contact profilometer) is used, it takes a day or longer until the three-dimensional shape data of one tooth row (for example, of the upper tooth row) is acquired.

The superposition of jaw movement data and tooth row shape data in (c) can also be adequately performed by using a commercially available software at present, by selecting an appropriate coordinate system and data format.

However, there is a problem with the simulation display in (d). That is to say, in the case of analysis at the time of functioning, such as mastication or the like, determination of occlusion of upper and lower tooth rows is important, and the determination of occlusion has been conventionally performed according to the method as follows.
(1) Based on a relative position of the upper jaw and the lower jaw obtained from jaw movement data, the positions of the upper tooth row and the lower tooth row are fixed (a set jaw position).
(2) For each surface point of the teeth of the upper tooth row in the set jaw position, distances from all surface points of the teeth of the lower tooth row are measured.
(3) The shortest distance among the above distances is set as the distance between the surface points of the upper tooth row from the lower tooth row.
(4) The above is calculated with respect to all surface points of the upper tooth row, and the shortest distance from the lower tooth row is set as the distance between the upper and lower tooth rows in the set jaw position.

In the above method (hereinafter, the method is called "a round-robin method"), in the calculation in (4), the amount of calculation becomes huge as the amount of data constituting the upper and lower tooth row shape increases, which results in a hindrance to quick analysis.

The present invention has been made to solve such conventional problems, and provides a method for quickly acquiring three-dimensional shape data of upper and lower tooth rows together with jaw movement data, first. The present invention also provides a method for calculating a distance between the upper and lower tooth rows at a high speed from the three-dimensional shape data of the upper and lower tooth rows which are thus acquired. Further, the present invention provides a method for performing simulation display of three-dimensional shapes of the upper and lower tooth rows and an occlusion contact region at a high speed based on technology behind these methods. In addition, the present invention provides an apparatus for carrying out these methods.

### SOLUTION TO PROBLEM

An upper and lower tooth row simulation display apparatus according to the present invention is defined in independent claim 1. Further embodiments of the apparatus are defined in dependent claims 2 to 4.

An upper and lower tooth row simulation display method that can be realized by the apparatus includes the steps of:
a) fitting a jaw movement sensor to an examinee, and acquiring jaw movement data on jaw movement of the examinee;
b) inserting an impression plate between the upper tooth row and the lower tooth row of the examinee to which the jaw movement sensor is fitted, the impression plate including a rigid flat plate having a top surface and a bottom surface each coated with an impression material, and guiding the examinee to perform a temporary occlusion;
c) Measuring impressions left on the impression material and a gauge mark provided on the rigid flat plate by using a three-dimensional shape measuring instrument, and thereby acquiring three-dimensional shape data of the upper tooth row, three-dimensional shape data of the lower tooth row and gauge mark data; and
d) performing simulation display of movement of the upper tooth row and the lower tooth row of the examinee, based on the three-dimensional shape data of the upper tooth row, the three-dimensional shape data of the lower tooth row and the gauge mark data, and jaw position data at a time of the temporary occlusion, and the jaw movement data.

In the simulation display method, the impression plate in which the top surface and bottom surface are coated with the impression material is inserted between the upper and lower tooth rows, and the examinee is guided to bite the impression plate (this is called temporary occlusion). Therefore, impressions of the upper tooth row and the lower tooth row can be acquired at a time. Further, a male model is not formed (reproduced) from the impressions on the impression material as in the prior art, but a three-dimensional shape, that is, the surface contour is acquired directly from the impression (a female mold), and therefore, acquisition of the three-dimensional shape data can be performed quickly. The three-dimensional shape data of the tooth rows are usually described in an STL (STereo Lithography) format that expresses by a set of facets which are triangular polygons each defined by the coordinates of three vertexes and a normal line vector.

When the three-dimensional shape data of the upper and lower tooth rows are acquired, the three-dimensional position data of the gauge mark provided on the rigid flat plate of the impression plate (when the gauge mark is large (i.e., a gauge mark body), three-dimensional shape data may be acquired) are also acquired at the same time. By matching the position data of the gauge mark (in the case of the shape data of the gauge mark body, the shape of the gauge mark body is known, and therefore, it is possible to determine the reference position data), the relative position of the shape data of the upper and lower tooth rows is determined, and the relative position of the upper and lower tooth rows at the time of temporary occlusion can be reproduced.

When the three-dimensional shape data of the upper and lower tooth rows are acquired, the jaw movement sensor is fitted to the examinee. The jaw movement sensor in this case refers to the sensor capable of acquiring the data of the relative movement of upper and lower jaws in six degrees of freedom, and devices described in Patent Literatures 1 to 4, for example, or the like can be used.

Besides acquiring the three-dimensional data of the upper and lower tooth rows, data of the jaw movements (border movements, masticatory movement or the like) of the examinee are acquired. The acquisition of the jaw movement data may be performed before or after the acquisition of the three-dimensional data of the upper and lower tooth rows. In general, data is acquired by starting the jaw movement from an intercuspal position (a state where the upper and lower tooth rows contact most). The jaw movement data is measured at a sampling rate (100 Hz or higher in actual) necessary to analyze the jaw movement, and is stored as a 4x4 transformation matrix of the coordinate system set for the lower jaw relative to the coordinate system set for the upper jaw, which will be described later.

The relative position of the upper and lower jaws detected by the jaw movement sensor at the time point of the temporary occlusion can be regarded as one point in the movement data of the upper and lower jaws. The relative position of the upper and lower tooth rows at this time point is already determined as described above. Accordingly, the above described acquired three-dimensional shape data of the upper and lower tooth rows are placed in the position, and based on the position, the upper and lower tooth rows are relatively moved by using the jaw movement data, whereby simulation display of the movements of the upper and lower tooth rows can be performed.

The upper and lower tooth row simulation display apparatus includes:
a) a jaw movement sensor that is fitted to an examinee, and acquires jaw movement data on jaw movement of the examinee,
b) an impression plate to be inserted between the upper tooth row and a lower tooth row of the examinee to which the jaw movement sensor is fitted, the impression plate including a rigid flat plate having a top surface and a bottom surface each coated with an impression material, and a gauge mark,
c) a three-dimensional shape measuring instrument for performing three-dimensional shape measurement of impressions left on the impression material by guiding the examinee to perform a temporary occlusion on the impression plate and the gauge mark, and thereby acquiring three-dimensional shape data of the upper tooth row, three-dimensional shape data of the lower tooth row and gauge mark data, and
d) a simulation processing section that displays simulation display of movement of the upper tooth row and the lower tooth row of the examinee, based on the three-dimensional shape data of the upper tooth row, the three-dimensional shape data of the lower tooth row and the gauge mark data, and jaw position data at a time of the temporary occlusion and the jaw movement data.

The jaw movement sensor may include:
a triaxial excitation coil fixed to the upper jaw or the lower jaw,
an AC power supply that provides an AC current to each of axial coils of the triaxial excitation coil,
a triaxial detection coil fixed to a jaw opposite to the jaw to which the triaxial excitation coil is fixed, and
a detection circuit that detects an induction current induced in each of the axial coils of the triaxial detection coil.

In the method described above, the part of the upper and lower tooth row three-dimensional shape data acquiring method extracted below can quickly acquire the three-dimensional shape data of the upper and lower tooth rows, and therefore it is useful by itself.

The upper and lower tooth row three-dimensional shape data acquiring method includes the steps of:
a) inserting an impression plate between the upper tooth row and the lower tooth row of an examinee, the impression plate including a rigid flat plate having a top surface and a bottom surface each coated with an impression material, and guiding the examinee to perform a temporary occlusion, and
b) acquiring three-dimensional shape data of the upper tooth row and three-dimensional shape data of the lower tooth row respectively by measuring impressions left on the impression material by using a three-dimensional shape measuring instrument.

A step of acquiring gauge mark data which is common to the upper and lower tooth rows as described before may be added to the acquiring method.

As described above, in dental examination and treatment, and in dental technique, it is necessary to determine a bite (occlusion) position of the upper and lower teeth accurately. However, in the simulation display of the upper and lower tooth rows described above, movements of the upper and lower tooth rows can be reproduced, but the occlusal position of the upper and lower tooth rows cannot be determined. In order to determine the occlusal position, a point where the three-dimensional shape data of the upper tooth row and the three-dimensional data of the lower tooth row come closer to each other than a predetermined occlusion reference value (i.e. a contact point) has to be determined. For the point, calculation has been conventionally performed by a round-robin method. When the inventors of the present application actually performed calculation according to the method by using the upper and lower tooth row shape data of a male adult, it took as long as about 1200 seconds.

Therefore, the inventors of the present application invented a method for performing the calculation at a higher speed with respect to the above problem. The method uses a program (an upper and lower tooth row simulation display program) that performs simulation display of the upper tooth row and the lower tooth row of an examinee on a screen based on the three-dimensional shape data of the upper tooth row and the three-dimensional shape data of the lower tooth row of the examinee, and includes the steps of:
a) carrying out a transformation of a coordinate system for setting a reference plane of the upper tooth row so as to be parallel with the display plane of the simulation display program, and
b) calculating, for each surface point in the upper tooth row, a distance between a corresponding surface point of the lower tooth row and the surface point of the upper tooth row having the same coordinate value in a plane parallel with the display plane.

The upper and lower tooth row distance high-speed calculation method described above can also be applied to three-dimensional shape data acquired by any method, independent of the method for acquiring the three-dimensional shape data of the upper and lower tooth rows.

Here, in the calculation of "a distance between a corresponding surface point of the lower tooth row and the surface point of the upper tooth row having the same coordinate value in a plane parallel with the display plane" in b), a function for acquiring a distance in a depth direction from a display screen included in the simulation display program can be used.

Accordingly, as the simulation display program which is used in the method, various generally-used programs can be used as long as the programs include the function that acquires the distance in the depth direction from the display screen.

In this method, a reference coordinate system of the upper tooth row (The reference coordinate system of the upper tooth row can be determined based on the three-dimensional shape data of the upper tooth row. The reference coordinate system of the upper tooth row is normally determined as an occlusion plane coordinate system which is the same as a reference coordinate system of jaw movement. Details will be described later.) is matched with a plane parallel with the display plane of the upper and lower tooth row simulation display program. The reference coordinate system of the upper tooth row may be matched with the display plane itself.

The lower tooth row is placed in a jaw position for determining an occlusion contact site (an occlusal contact region) by using the jaw movement data of the examinee according to the upper and lower tooth row simulation display program. The position of the upper and lower tooth rows in the above described temporary occlusion may be used. In this position of the lower jaw, the calculation in the aforementioned b) is performed. If the display plane is set as an xy-plane, in b), for each surface point (obtained as the center of gravity of the facet) in the upper tooth row, the distance from the surface of the lower tooth row having the same xy-coordinate value is calculated, and this is calculation of a distance (a depth) in a z-axis direction, and therefore is extremely easy. When the reference plane (the occlusion plane) of the upper tooth row is matched with the display plane, this is merely the z-coordinate of the corresponding surface point in the lower tooth row. Further, there is no more than one distance for each of the surface points in the upper tooth row.

Based on the distance between the upper and lower tooth rows, the occlusal contact regions of the upper and lower tooth rows can be determined as follows:
(1) First, in the present jaw position, for each of the facets configuring the surface of the upper tooth row, the difference between the z-coordinate of the facet and the z-coordinate of the corresponding surface of the lower tooth row is obtained as the distance d from the lower tooth row. The value of the distance is stored in correspondence with each of the facets in the STL format.
(2) (A set of) facets of the upper and lower tooth rows where the distance d between the upper and lower tooth rows satisfies [d≤d0] with respect to a predetermined occlusion reference value d0 is determined as an occlusal contact region. Recent study shows that the precision which a living body requires to the occlusion surface shape is about 20 µm, but considering actual clinical sensation such as tooth mobility, the occlusion reference value d0 is preferably set at approximately 200 µm in reality (Non Patent Literature 4).

### ADVANTAGEOUS EFFECTS OF INVENTION

One example of application of the apparatus according to the present invention is to provide information such as the jaw position and occlusion contact site at the time of manifestation of bruxism which has not been provided so far. Further, application can be made to an everyday dental clinical practice such as occlusal equilibration and a masticatory function test; it can be expected that, when a proper biting action cannot be performed, the spot to be scaled and the spot to be built up become quite obvious. Ultimately, dental treatment can be expected to be able to contribute more to health and longevity of a nation.

### BRIEF DESCRIPTION OF DRAWINGS

Fig. 1 is a schematic configuration diagram of an upper and lower tooth row three-dimensional simulation display system that is one example of the present invention.
Fig. 2 is a schematic flowchart of processing in the embodiment.
Fig. 3A is a schematic side view showing a method for acquiring impressions of upper and lower tooth rows of an examinee in the embodiment, and Fig. 3B is a perspective view of an impression plate used in the method.
Fig. 4A is a top view of the impression plate, and Fig. 4B is a bottom view of the impression plate.
Fig. 5 is a front view of a three-dimensional shape measuring instrument used in the embodiment.
Fig. 6A is a view of a three-dimensional shape of the upper tooth row including gauge mark spheres which is acquired by measuring a top surface of the impression plate, and Fig. 6B is a view of a three-dimensional shape of the lower tooth row including gauge mark spheres.
Fig. 7 is a view of display of a three-dimensional shape of the upper and lower tooth rows in a state of temporary occlusion at a time of acquiring the impressions.
Fig. 8 is a view of display of the three-dimensional shapes of the upper and lower tooth rows in an arbitrary jaw position.
Fig. 9 is a diagram of a data structure of data in an STL format which is adopted to express the three-dimensional data of the tooth rows.
Fig. 10 is an explanatory view of an occlusal surface coordinate system.
Fig. 11 is a view of the three-dimensional shape of the upper tooth row with a reference plane matched with a window plane of OpenGL.
Fig. 12A and 12B are schematic explanatory views of methods for calculating a distance between the upper and lower tooth rows of a conventional method and a method of the present invention, respectively.
Fig. 13 is a view showing the three-dimensional shapes of the upper and lower tooth rows in an occlusal state.
Fig. 14A and Fig. 14B are views visually displaying occlusal contact regions of the upper and lower tooth rows, Fig. 14A shows the view in a case of calculating by the conventional method, and Fig. 14B shows the view in a case of calculating by the method of the present invention.

### DESCRIPTION OF EMBODIMENT

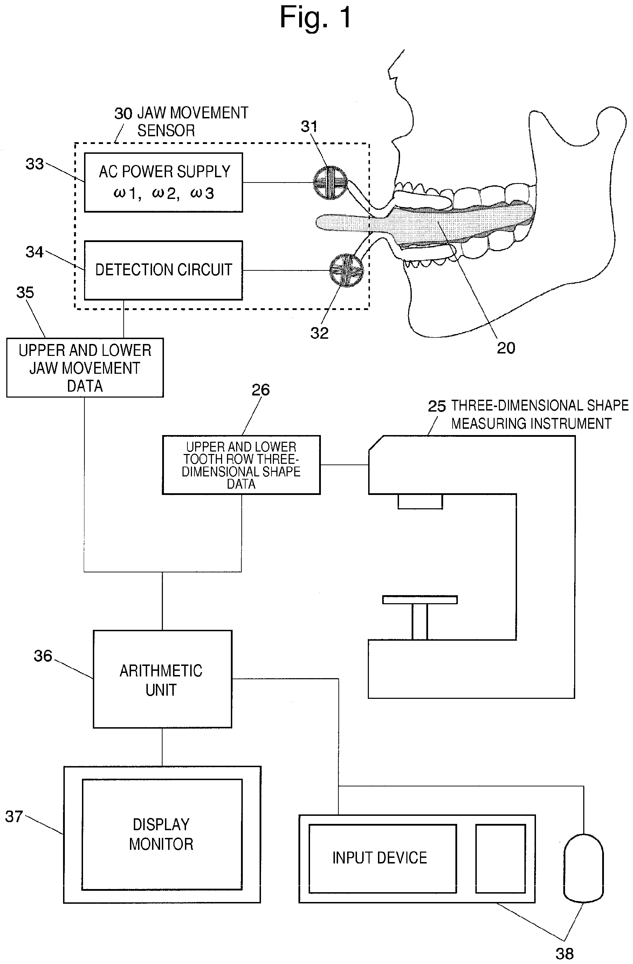

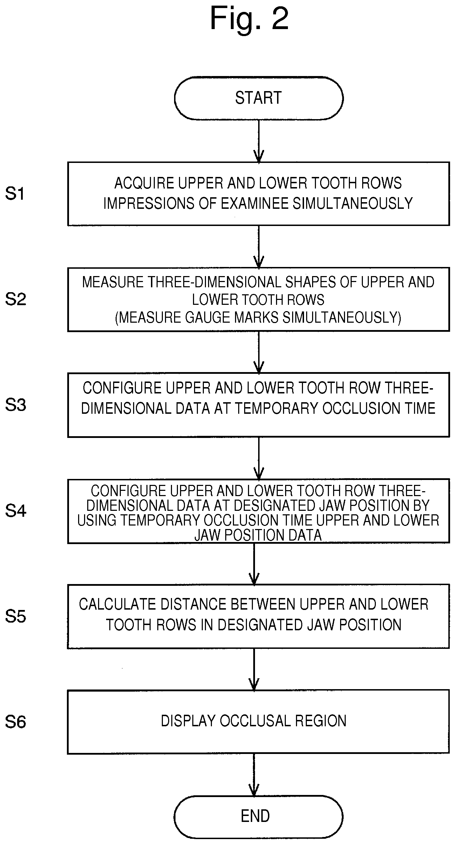

One embodiment of the present invention will be described based on a system schematic configuration diagram in Fig. 1 and a flowchart in Fig. 2.

As shown in Fig. 1, a system is constituted of a jaw movement sensor 30 for detecting a jaw position and jaw movement of an examinee, an impression plate 20 for simultaneously acquiring three-dimensional shapes of upper and lower tooth rows of the examinee, a three-dimensional shape measuring instrument 25 that generates three-dimensional shape data of the upper and lower tooth rows from the acquired impression plate 20, an arithmetic unit 36 that performs various arithmetic operations based on the acquired upper and lower jaw movement data 35 and the acquired upper and lower tooth row three dimensional shape data 26.

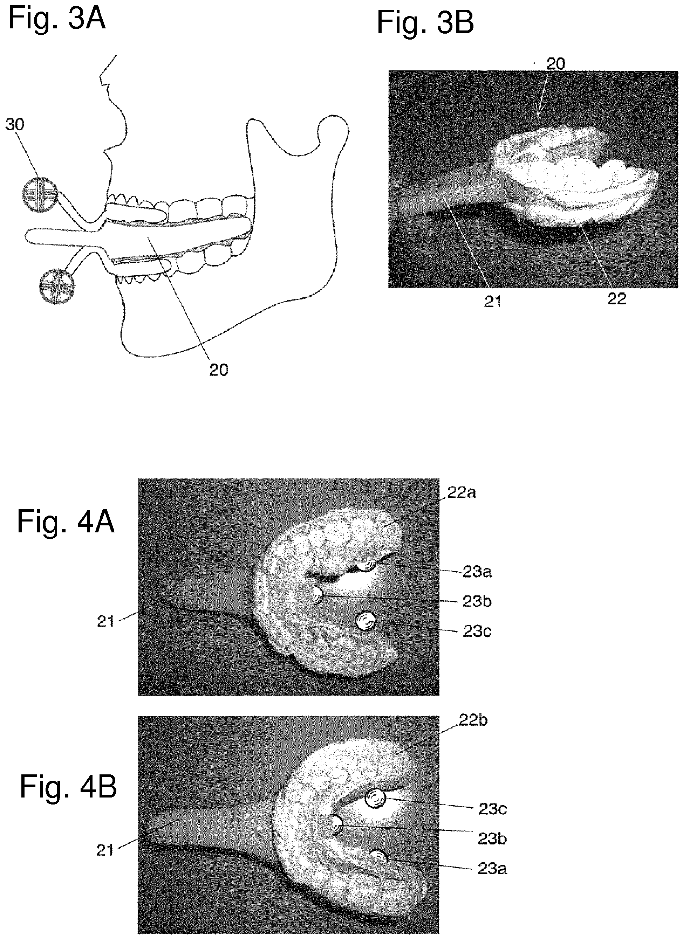

First, in order to measure three-dimensional shapes of the upper and lower tooth rows of an examinee, impressions of the upper and lower tooth rows are simultaneously acquired (step S1). More specifically, as shown in Fig. 3A, the impression plate 20 is inserted between the upper and lower tooth rows of the examinee, and the examinee is guided to bite the impression plate 20 ("temporary occlusion"). As shown in Fig. 3B, an impression material 22 such as dental silicone is applied on top and bottom surfaces of a U-shaped tray 21 of the impression plate 20. Thereby, the impressions of the upper and lower tooth rows of the examinee can be acquired at a time.

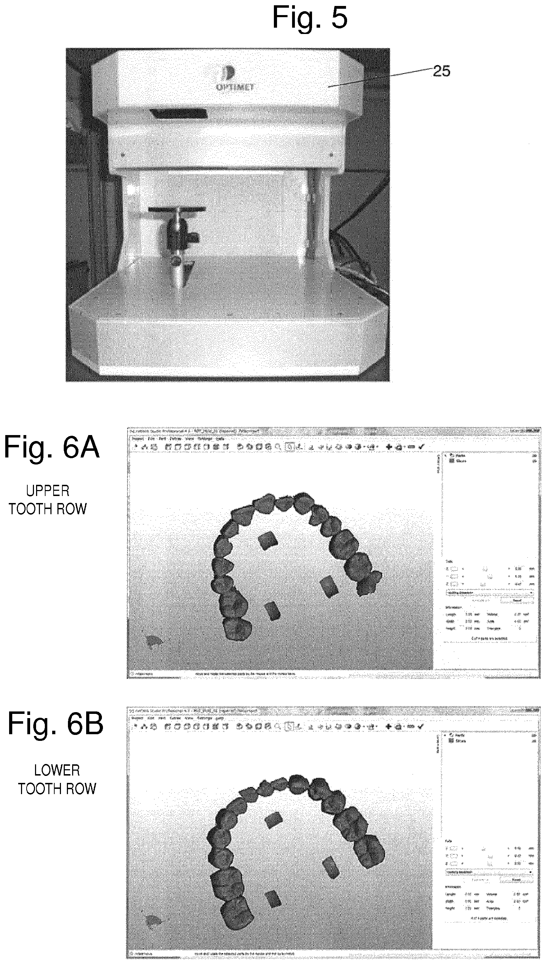

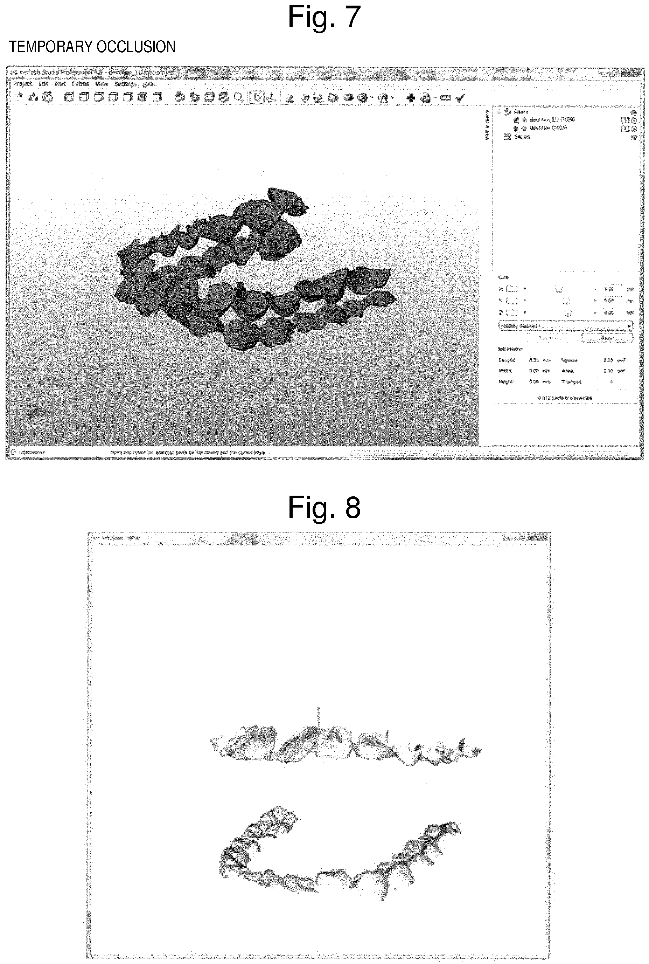

Three-dimensional shape data on the surfaces of the impression plate 20 are acquired by the three-dimensional shape measuring instrument 25 as shown in Fig. 5 (step S2). The top surface and the bottom surface of the impression plate 20 are independently subject to the process by the three-dimensional shape measuring instrument 25 so that the three-dimensional shape data of the upper tooth row, and the three-dimensional shape data of the lower tooth row are separately acquired. The both shape data are matched with each other as follows. That is to say, as shown in Fig. 4A and Fig. 4B, three gauge mark spheres 23a, 23b and 23c are previously fixed to the tray 21 of the impression plate 20, and three dimensional shapes of the gauge mark spheres 23a, 23b and 23c in the respective top and the bottom surfaces are also measured simultaneously with respective impression materials 22a and 22b (Fig. 6A and Fig. 6B). Each of the three-dimensional shape data of the upper and lower tooth rows acquired in this manner has the three-dimensional data of the three gauge mark spheres 23a, 23b and 23c. Setting a positional relation of both data of upper and lower tooth rows so that each data of the three gauge mark spheres represents spherical shape, the three-dimensional data of the upper and lower tooth rows are matched with each other and integrated (step S3, Fig. 7).

When the impressions of the upper and lower tooth rows are acquired with the impression plate 20, the jaw movement sensor 30 for detecting positions of the upper and lower jaws is fitted to the examinee, as shown in Fig. 1 and Fig. 3A. At the time of the temporary occlusion, a relative position of the upper and lower jaws (a position of the lower jaw relative to the upper jaw) is detected. The jaw position is set as j1. The jaw position j1 is data having six elements corresponding to six degrees of freedom that will be described later.

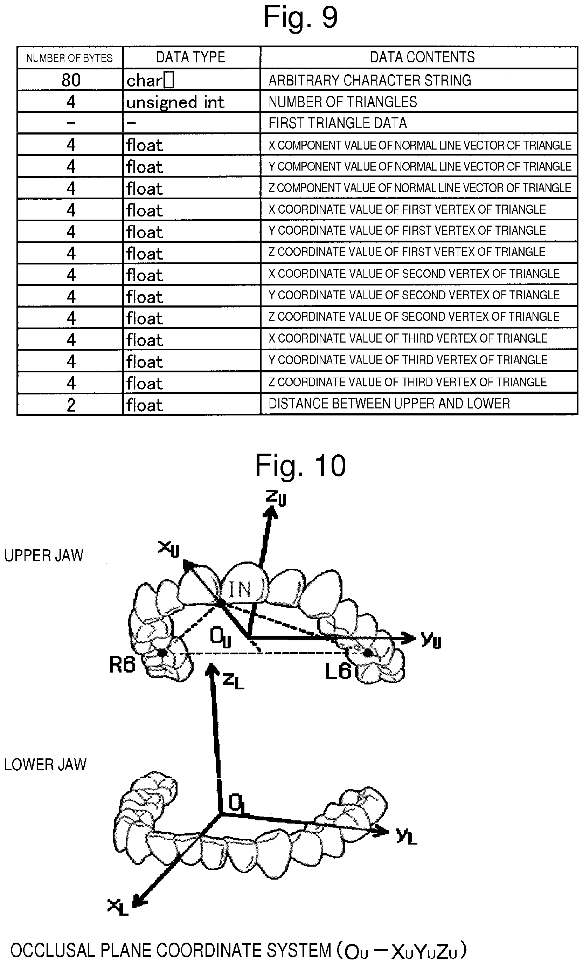

Apart from acquisition of the impressions with the impression plate 20, the examinee to which the jaw movement sensor 30 is fitted is guided to variously move own jaw, and relative positions (positions of the lower jaw relative to the upper jaw) and movements of the upper and lower jaws during the movement are measured in six degrees of freedom (an x-coordinate value, a y-coordinate value, a z-coordinate value, an x-axis rotation, a y-axis rotation and a z-axis rotation). Various methods can be adopted, and as one example, a specific method is, as shown in Fig. 1 to apply AC currents ω1, ω2 and ω3 respectively from an AC power supply 33 to respective axis coils of a triaxial (xe, ye and ze axes) excitation coil 31 fixed to the upper jaw so that a detection circuit 34 detects induced currents induced by the AC currents in respective axis coils of a triaxial (xi, yi and zi axes) detection coil 32 fixed to the lower jaw. Naturally, the fixed positions of the excitation coil 31 and the detection coil 32 may be reversed. A detailed configuration and operation of the jaw movement sensor 30 used in the method are described in detail in Patent Literatures 2 to 4. In the coordinate systems of the upper and lower jaws, as shown in Fig. 10, a plane including three gauge marks on the upper jaw tooth row (an incisal point IN, a left side first molar central fossa L6 and a right side first molar central fossa R6) is set as a reference plane (occlusal surface), a median point of the three points is defined as an origin 0, a straight line connecting the origin 0 and the incisal point IN is defined as an X-axis, a normal line of the occlusal surface passing through the origin is defined as a Z-axis, and a straight line perpendicular to both the axes is defined as a Y-axis. As shown in Fig. 10, an occlusal surface coordinate system (0_{U}-X_{U}Y_{U}Z_{U}) is set on the upper jaw, and coincides with a coordinate system (0_{L}-X_{L}Y_{L}Z_{L}) that is set on the lower jaw in an intercuspal position (a state where the upper and lower tooth rows bite) (Non Patent Literature 3).

The data of the various upper and lower jaw positions thus acquired is stored in a predetermined storage region 35 as a 4x4 transformation matrix of the coordinate system set on the lower jaw relative to the coordinate system set on the upper jaw.

The three-dimensional shape data of the upper and lower tooth rows which is configured in the aforementioned step S3 and stored in the storage region 26 is data in the jaw position j1 in temporary occlusion. Thus, three-dimensional shape data of the upper and lower tooth rows in a jaw position j2 other than the jaw position j1 is configured as follows. First, an inverse matrix T_{L>U} of the jaw movement data (4x4matrix) in the jaw position j1 is created. The transformation matrix T_{L>U} is a 4x4matrix. Next, the three-dimensional data of the lower tooth row in the jaw position j1 is multiplied by the transformation matrix T_{L>U}, and three-dimensional data of the lower tooth row in the intercuspal position is configured. By combining the three-dimensional data of the lower tooth row and the three-dimensional data of the upper tooth row, the three-dimensional data of the upper and lower tooth rows in the intercuspal position are configured (step S4). In this manner, the coordinate systems of the jaw movement data and the three-dimensional data of the upper and lower tooth rows can be caused to coincide with each other, and the three-dimensional data of the lower tooth row in the intercuspal position is multiplied by the jaw movement data (4x4 transformation matrix), whereby the three-dimensional shape data of the upper and lower tooth rows in the arbitrary jaw position j2 of the examinee can be configured. That is to say, arbitrarily instruction by an operator with an input device 38 cause a display monitor 37 to display, the three-dimensional shapes of the upper and lower tooth rows in the instructed position are displayed (Fig. 8). By preparing data in each jaw position in advance, or by using a high-speed arithmetic unit, movement of the upper and lower tooth rows can also be simulated.

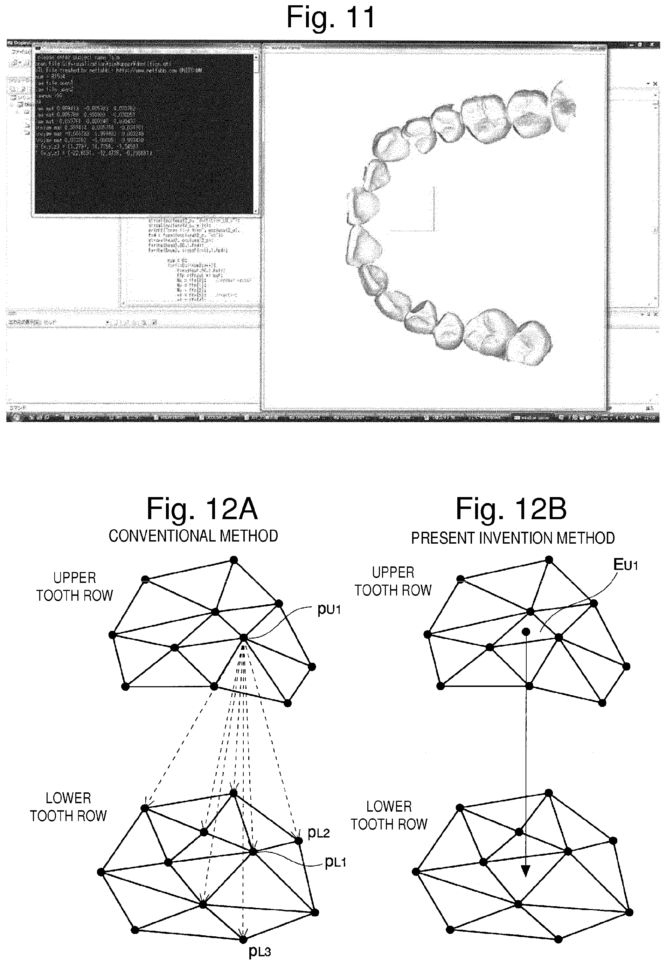

In the simulation display of the upper and lower tooth rows as above, it is necessary to determine occlusion of the upper and lower tooth rows. Further, in pathological judgment of an examinee (a patient) based on the three-dimensional shape data, determination of an occlusal position is important. An occlusal position can be determined as a region where the upper and lower tooth rows are closer to each other than an occlusion reference value, and an occlusal position has been conventionally calculated by a round-robin method, as shown in Fig. 12A. That is to say, with respect to a certain point pU1 in the upper tooth row, distances to respective points pL1, pL2, pL3, ... in the lower tooth row are calculated, and the smallest value among them is set as a distance from the point pU1 of the upper tooth row to the entire lower tooth row. Such a calculation is performed for each point in the upper tooth row, and a distance from each point to the lower tooth row is set as a distance d_{UL}. Such a calculation is performed for each jaw position, and a region where the distance d_{UL} between the upper and lower tooth rows is smaller than the occlusion reference value is determined as an occlusal contact region.

As above, in the conventional method, there is a problem that the amount of calculation becomes huge as the number of points in the three-dimensional shape data of the tooth rows increases, and the calculation time period becomes longer. For example, when the three-dimensional shapes of the upper and lower tooth rows are respectively configured by 81504 triangular facets in the upper jaw and 67704 triangular facets in the lower jaw, approximately 1200 seconds for one jaw is required for calculating the distance between the upper and lower tooth rows.

For this problem, the present inventors have developed a method capable of determining an occlusal position at a high speed by using three-dimensional shape display software (three-dimensional simulation software) which is generally available. First, the present inventors have used OpenGL (trademark) that is a cross-platform API (Programming Interface) which is opened to the public as open specifications and is usable in UNIX, PC UNIX, Windows, Mac OS X (are all registered) and the like, in simulation display of upper and lower tooth rows three-dimensional shapes. In this program, three-dimensional polygons can be rendered, and therefore reading and rendering in the binary format of a STL format are possible. The data structure is as shown in Fig. 9. Normal line vectors (three) of the triangular facet are shown first, and next, respective coordinates (3 by 3 = 9) of the triangular facet are shown in sequence of X/Y/Z. Thereafter, unused data of two bytes follows. Most of software programs do not evaluate this part, and therefore, zero is usually shown in this part, but the three-dimensional shape data of the upper and lower tooth rows of the present embodiment stores the distance d between the opposite jaw surfaces. The above described three-dimensional shape data of the upper and lower tooth rows are converted into a STL format, and is taken into OpenGL, whereby the three-dimensional shapes of the upper and lower tooth rows can be displayed on a display screen of OpenGL at will. For various kinds of calculation and the like, Visual C++ (manufactured by Microsoft Corporation) is used.

The distance between the upper and lower tooth rows is calculated by using a three-dimensional shape depth information (depth value) acquisition function of the program. That is to say, first of all, coordinates of the upper and lower tooth row three-dimensional shape data (object coordinates) which are taken in are transformed into coordinates of OpenGL (window coordinates) (<gluProject> function). At this time, the reference plane of the upper tooth row is caused to coincide with a window plane of OpenGL (Fig. 11). The reference plane of the upper tooth row refers to a plane (an XY-plane) including the three gauge marks on the upper jaw tooth row (the incisal point IN, the left side first molar central fossa L6, and the right side first molar central fossa R6), in the aforementioned upper and lower jaws coordinate systems (Fig. 10). Next, depth information (a depth value) of a pixel of the lower tooth row three-dimensional shape data in the window coordinate value to (x, y) coordinate values of the median point of the first facet of the triangular polygons composing the surface three-dimensional shape of the upper tooth row is acquired (<glReadPixels> function). The window coordinates of the corresponding points of the three-dimensional shape of the lower tooth row which are thus acquired are transformed into the object coordinates (<gluUnProject> function). A difference between the z-coordinate of the median point of the first facet of the triangular facets composing the surface three-dimensional shape of the upper tooth row and the depth value is obtained. This means a measurement of a distance between the median point of one facet E_{U1} in the upper tooth row and the facet in the corresponding position in the lower tooth row, as shown in Fig. 12B. This is set as a distance between the facet and the lower tooth row. All the triangular facets in the upper tooth row are subject to the distance calculation (step S5). The distance of the upper tooth row from the lower tooth row is similarly obtained. When the distance between the upper and lower tooth rows is calculated with respect to all the facets by the present method, a calculation time period is approximately three seconds for one jaw, and is significantly shortened as compared with 1200 seconds which is required in the case of the conventional method (the service system: Dell (R) Precision M6400/Intel (R) Core2 Duo 2.53GHz/Windows (R) XP Professional X64 Edition/Microsoft Visual C++).

In this manner, the distance between the upper and lower tooth rows can be calculated in a short period of time in an optional jaw position.

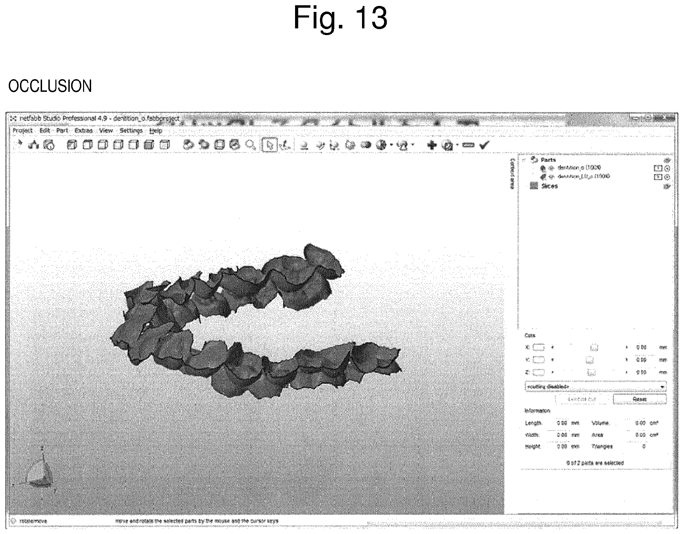

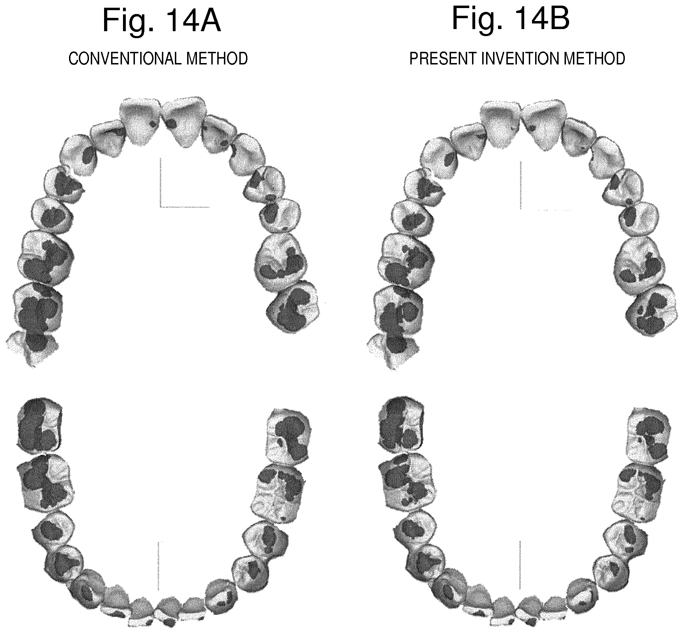

In the jaw position in an occlusal state (Fig. 13), a set of the points having values equal to or smaller than a predetermined threshold value (The Occlusion reference value. In reality, approximately 200 µm.), of the distances between the upper and lower tooth rows form an occlusal contact region. Fig. 14A and Fig. 14B shows visual display of the occlusal contact regions (step S6). Fig. 14A is a display of occlusal contact regions by calculating the distances between the upper and lower tooth rows according to the aforementioned conventional method, and Fig. 14B is a display of occlusal contact regions by calculating the distances according to the method according to the present invention, and the occlusal contact regions of both of them substantially coincide with one another.

In the above described embodiment, OpenGL is used in simulation display of the three-dimensional shapes, but other software programs having similar functions such as DirectX (Microsoft Corporation) can also be used.

### REFERENCE SIGNS LIST

20 ... Impression Plate
21 ... Tray
22, 22a, 22b ... Impression Material
23a, 23b, 23c ... Gauge Mark Sphere
25 ... Three-dimensional Shape Measuring Instrument
26 ... Upper And Lower Tooth Row Three-dimensional Shape Data (Storage Region)
30 ... Jaw Movement Sensor
31 ... Excitation Coil
32 ... Detection Coil
33 ... AC Power Supply
34 ... Detection Circuit
35 ... Upper and Lower Jaw Movement Data (Storage Region)
36 ... Arithmetic Unit
37 ... Display monitor
38 ... Input Device

## Claims

1. An upper and lower tooth row simulation display apparatus, comprising:
a) a jaw movement sensor (30) that is fitted to an examinee, and acquires jaw movement data (35) on jaw movement of the examinee;
b) an impression plate (20) to be inserted between an upper tooth row and a lower tooth row of the examinee to which the jaw movement sensor (30) is fitted, the impression plate (20) including a rigid flat plate having a top surface and a bottom surface each coated with an impression material (22, 22a, 22b), and three gauge marks (23a, 23b, 23c);
c) a three-dimensional shape measuring instrument (25) for performing three-dimensional shape measurement of impressions left on the impression material (22, 22a, 22b) by the examinee performing a temporary occlusion on the impression plate (20), and the three gauge marks (23a, 23b, 23c), and thereby acquiring three-dimensional shape data (26) of the upper tooth row with three gauge mark data, and three-dimensional shape data (26) of the lower tooth row with three gauge mark data, separately; and
d) a simulation processing section (36) that performs simulation display of movement of the upper tooth row and the lower tooth row of the examinee, based on the three-dimensional shape data (26) of the upper tooth row, the three-dimensional shape data (26) of the lower tooth row and the gauge mark data, and jaw position data at a time of the temporary occlusion and the jaw movement data (35).

2. The upper and lower tooth row simulation display apparatus according to claim 1,
wherein the jaw movement sensor (30) comprises:
a triaxial excitation coil (31) fixed to an upper jaw or a lower jaw,
an AC power supply (33) that provides an AC current to each of axial coils of the triaxial excitation coil (31),
a triaxial detection coil (32) fixed to a jaw opposite to the jaw to which the triaxial excitation coil (31) is fixed, and
a detection circuit (34) that detects an induction current induced in each of axial coils of the triaxial detection coil (32).

3. The upper and lower tooth row simulation display apparatus according to claim 1 or 2, further comprising:
e) a reference plane setting section that performs processing of setting a reference plane of the upper tooth row so as to be parallel with a display plane of a simulation display program by using the program that performs simulation display of the upper tooth row and the lower tooth row of the examinee on a screen (37) based on the three-dimensional shape data (26) of the upper tooth row and the three-dimensional shape data (26) of the lower tooth row of the examinee; and
f) a distance calculating section that calculates, for each surface point in the upper tooth row, a distance between the upper and lower tooth rows that is a distance between a corresponding surface point in the lower tooth row and the surface point in the upper tooth row having the same coordinate value in a plane parallel with the display plane.

4. The upper and lower tooth row simulation display apparatus according to claim 3, further comprising:
g) an occlusal state setting section that brings the three-dimensional shape data (26) of the upper tooth row and the three-dimensional shape data (26) of the lower tooth row close to each other based on the jaw movement data (35) of the examinee, and sets the upper and lower tooth rows to an occlusal state; and
h) an occlusal contact region determining section that determines surface points of the upper and lower tooth rows where the distances between the upper and lower tooth rows are within a range of a predetermined occlusion reference value as an occlusal contact region, in the occlusal state.

## Patentansprüche

1. Obere-und-Untere-Zahnreihe-Simulationsanzeigevorrichtung, umfassend:
a) einen Kieferbewegungssensor (30), der an einem Prüfling angebracht ist und Kieferbewegungsdaten (35) über die Kieferbewegung des Prüflings erfasst;
b) eine Abdruckplatte (20), die zwischen einer oberen Zahnreihe und einer unteren Zahnreihe des Prüflings einzuführen ist, an dem der Kieferbewegungssensor (30) angebracht ist, wobei die Abdruckplatte (20) eine starre flache Platte mit einer Oberseite und einer Unterseite, die jeweils mit einem Abdruckmaterial (22, 22a, 22b) beschichtet sind, und drei Messmarken (23a, 23b, 23c) aufweist;
c) ein Dreidimensionale-Form-Messgerät (25) zum Ausführen einer Messung der dreidimensionalen Form von Abdrücken, die durch den Prüfling, der eine temporäre Okklusion auf der Abdruckplatte (20) durchführt, auf dem Abdruckmaterial (22, 22a, 22b) zurückgelassen wurden, und der drei Messmarken (23a, 23b, 23c), und dadurch separates Erfassen von dreidimensionalen Formdaten (26) der oberen Zahnreihe mit Daten von drei Messmarken und von dreidimensionalen Formdaten (26) der unteren Zahnreihe mit Daten von drei Messmarken; und
d) einen Simulationsverarbeitungsabschnitt (36), der eine Simulationsanzeige der Bewegung der oberen Zahnreihe und der unteren Zahnreihe des Prüflings basierend auf den dreidimensionalen Formdaten (26) der oberen Zahnreihe, den dreidimensionalen Formdaten (26) der unteren Zahnreihe und den Daten der Messmarken, sowie den Kieferpositionsdaten zu einem Zeitpunkt der temporären Okklusion und den Kieferbewegungsdaten (35) durchführt.

2. Obere-und-Untere-Zahnreihe-Simulationsanzeigevorrichtung nach Anspruch 1,
wobei der Kieferbewegungssensor (30) umfasst:
eine dreiachsige Erregerspule (31), die an einem Oberkiefer oder einem Unterkiefer angebracht ist,
eine Wechselstromversorgung (33), die jeder von Axialspulen der dreiachsigen Erregerspule (31) einen Wechselstrom zuführt,
eine dreiachsige Detektionsspule (32), die an einem Kiefer befestigt ist, der dem Kiefer gegenüberliegt, an dem die dreiachsige Erregerspule (31) befestigt ist, und
eine Detektionsschaltung (34), die einen Induktionsstrom detektiert, der in jeder von Axialspulen der dreiachsigen Detektionsspule (32) induziert wird.

3. Obere-und-Untere-Zahnreihe-Simulationsanzeigevorrichtung nach Anspruch 1 oder 2, des Weiteren umfassend:
e) einen Bezugsebenen-Einstellabschnitt, der eine Verarbeitung der Einstellung einer Bezugsebene der oberen Zahnreihe ausführt, um parallel zu einer Anzeigeebene eines Simulationsanzeigeprogramms zu sein, unter Verwendung des Programms, das die Simulationsanzeige der oberen Zahnreihe und der unteren Zahnreihe des Prüflings auf einem Bildschirm (37) basierend auf den dreidimensionalen Formdaten (26) der oberen Zahnreihe und den dreidimensionalen Formdaten (26) der unteren Zahnreihe des Prüflings durchführt; und
f) einen Abstandsberechnungsabschnitt, der für jeden Oberflächenpunkt in der oberen Zahnreihe einen Abstand zwischen der oberen und der unteren Zahnreihe berechnet, welches ein Abstand zwischen einem entsprechenden Oberflächenpunkt in der unteren Zahnreihe und dem Oberflächenpunkt in der oberen Zahnreihe mit dem gleichen Koordinatenwert in einer Ebene parallel zu der Anzeigeebene ist.

4. Obere-und-Untere-Zahnreihe-Simulationsanzeigevorrichtung nach Anspruch 3, des Weiteren umfassend:
g) einen Okklusionszustand-Einstellabschnitt, der die dreidimensionalen Formdaten (26) der oberen Zahnreihe und die dreidimensionalen Formdaten (26) der unteren Zahnreihe basierend auf den Kieferbewegungsdaten (35) des Prüflings nahe beieinander bringt und die obere und untere Zahnreihe auf einen Okklusionszustand einstellt; und
h) einen Okklusion-Kontaktbereich-Bestimmungsabschnitt, der Oberflächenpunkte der oberen und unteren Zahnreihen bestimmt, wobei die Abstände zwischen der oberen und unteren Zahnreihe in dem Okklusionszustand innerhalb eines Bereichs eines vorbestimmten Okklusionsbezugswerts als ein Okklusionskontaktbereich liegen.

## Revendications

1. Dispositif d'affichage de simulation de rangées de dents supérieure et inférieure, comprenant:
a) un capteur de mouvement de mâchoire (30) qui est monté sur un candidat et qui acquiert des données de mouvement de mâchoire (35) relatives au mouvement de mâchoire du candidat;
b) une plaque d'empreinte (20) à insérer entre une rangée de dents supérieure et une rangée de dents inférieure du candidat sur lequel ledit capteur de mouvement de mâchoire (30) est monté, la plaque d'empreinte (20) comprenant une plaque plate rigide ayant une face supérieure et une face inférieure qui sont revêtues chacune d'un matériau d'empreinte (22, 22a, 22b), et trois repères (23a, 23b, 23c);
c) un instrument de mesure de forme tridimensionnelle (25) pour réaliser une mesure de la forme tridimensionnelle d'empreintes laissées sur le matériau d'empreinte (22, 22a, 22b) par le candidat effectuant une occlusion temporaire sur la plaque d'empreinte (20), et des trois repères (23a, 23b, 23c), et acquérir ainsi de manière séparée des données de forme tridimensionnelle (26) de la rangée de dents supérieure avec des données de trois repères, et des données de forme tridimensionnelle (26) de la rangée de dents inférieure avec des données de trois repères; et
d) une section de traitement de simulation (36) qui réalise un affichage de simulation du mouvement de la rangée de dents supérieure et de la rangée de dents inférieure du candidat, sur la base des données de forme tridimensionnelle (26) de la rangée de dents supérieure, des données de forme tridimensionnelle (26) de la rangée de dents inférieure et des données des repères, ainsi que des données de position de mâchoire à un moment de l'occlusion temporaire et des données de mouvement de mâchoire (35).

2. Dispositif d'affichage de simulation de rangées de dents supérieure et inférieure selon la revendication 1,
dans lequel le capteur de mouvement de mâchoire (30) comprend:
une bobine d'excitation triaxiale (31) qui est fixée à une mâchoire supérieure ou à une mâchoire inférieure,
une alimentation en courant alternatif (33) qui fournit un courant alternatif à chacune de bobines axiales de la bobine d'excitation triaxiale (31),
une bobine de détection triaxiale (32) qui est fixée à une mâchoire opposée à la mâchoire à laquelle est fixée la bobine d'excitation triaxiale (31), et
un circuit de détection (34) qui détecte un courant d'induction induit dans chacune de bobines axiales de la bobine de détection triaxiale (32).

3. Dispositif d'affichage de simulation de rangées de dents supérieure et inférieure selon la revendication 1 ou 2, comprenant en outre:
e) une section de réglage de plan de référence qui effectue un traitement du réglage d'un plan de référence de la rangée de dents supérieure de manière à être parallèle à un plan d'affichage d'un programme d'affichage de simulation, en utilisant le programme qui effectue l'affichage de simulation de la rangée de dents supérieure et de la rangée de dents inférieure du candidat sur un écran (37) sur la base des données de forme tridimensionnelle (26) de la rangée de dents supérieure et des données de forme tridimensionnelle (26) de la rangée de dents inférieure du candidat; et
f) une section de calcul de distance qui calcule, pour chaque point de surface dans la rangée de dents supérieure, une distance entre les rangées de dents supérieure et inférieure qui est une distance entre un point de surface correspondant dans la rangée de dents inférieure et le point de surface dans la rangée de dents supérieure ayant la même valeur de coordonnées dans un plan parallèle au plan d'affichage.

4. Dispositif d'affichage de simulation de rangées de dents supérieure et inférieure selon la revendication 3, comprenant en outre:
g) une section de réglage d'état occlusal qui rapproche les données de forme tridimensionnelle (26) de la rangée de dents supérieure et les données de forme tridimensionnelle (26) de la rangée de dents inférieure, sur la base des données de mouvement de mâchoire (35) du candidat, et règle les rangées de dents supérieure et inférieure à un état occlusal; et
h) une section de détermination de zone de contact occlusal qui détermine des points de surface des rangées de dents supérieure et inférieure, les distances entre les rangées de dents supérieure et inférieure étant comprises, à l'état occulsal, dans une plage d'une valeur de référence d'occlusion prédéterminée en tant que zone de contact occlusal.
